(19) Europäisches Patentamt
European Patent Office
Office européen des brevets

(11) Numéro de publication : 0 459 893 A1

# (12) DEMANDE DE BREVET EUROPEEN

(21) Numéro de dépôt : 91401379.2

(51) Int. Cl.⁵ : B01D 67/00

(22) Date de dépôt : 29.05.91

(30) Priorité : 01.06.90 FR 9006865

(43) Date de publication de la demande : 04.12.91 Bulletin 91/49

(84) Etats contractants désignés : AT BE CH DE DK ES FR GB GR IT LI LU NL SE

(71) Demandeur : CYCLOPORE S.A.
Avenue Einstein 11 B
B-1348 Louvain-La-Neuve (BE)

(72) Inventeur : Haumont,Charles
Rue des Champs Elysées,84
B-1050 Bruxelles (BE)
Inventeur : Legras, Roger
Rue des Bourgmestres 6
B-4280 Lens Saint Rémy (Hannut) (BE)

(74) Mandataire : Warcoin, Jacques et al
Cabinet Régimbeau 26, avenue Kléber
F-75116 Paris (FR)

## (54) Matériau microperforé transparent et procédé de préparation.

(57) La présente invention a pour objet un matériau solide en feuille microperforé transparent à une lumière de longueurs d'ondes dans le visible ou l'infrarouge, caractérisé en ce que la distance moyenne entre deux perforations immédiatement voisines est d'au moins 5, de préférence 7 μm, et ce sur chacune des faces du matériau.

De préférence, l'angle d'inclinaison moyen des perforations dans l'épaisseur du matériau est inférieur à 10°, voire 5°.

Dans un mode de réalisation particulier, le matériau est une membrane réalisée à partir d'un film polymère flexible d'une épaisseur comprise entre 0,1 et 100 μ, plus généralement entre 5 et 50 μ, et les diamètres de perforation étant compris entre 0,01 et 15 μ.

Les membranes obtenues selon l'invention sont particulièrement utiles comme membranes support pour une lecture au microscope optique ou une spectroscopie infrarouge. Elles peuvent être utilisées aussi comme membranes de filtration ou encore comme support de culture cellulaire entre autres.

EP 0 459 893 A1

La présente invention concerne un matériau solide en feuille microperforé transparent à la lumière visible et aux longueurs d'ondes dans le domaine infrarouge.

En particulier, la présente invention concerne des membranes transparentes constituées d'un film de polymère transparent microperforé.

Enfin, la présente invention concerne un procédé de préparation dudit matériau ou desdites membranes.

On connait déjà l'obtention de perforations dans un matériau solide en feuille par réalisation, dans une première phase, de traces d'endommagement du matériau au moyen de particules énergétiques altérant le matériau le long de leurs trajectoires et, dans une seconde phase, par traitement chimique, pour attaquer sélectivement le matériau le long des traces d'endommagement.

Plusieurs variantes de mise en oeuvre de ce type de procédé ont été proposées dans l'état de la technique. On peut citer en particulier les brevets US 3 303 085, US 3 493 751, US 3 612 871 ainsi que le brevet GB 1 375 204. D'autre variantes de mise en oeuvre de procédé de ce type sont encore envisagées dans US 3 713 921, US 3 852 134, US 3 677 844 et FR 2 180 215.

On a décrit dans la demande de brevet international WO 87/05850 un procédé perfectionné de réalisation de microperforation dans un matériau solide en feuille de ce type, ainsi qu'un dispositif d'irradiation pour la mise en oeuvre de ce procédé.

Le procédé décrit dans WO 87/05850 permet de fabriquer un matériau perforé de très bonne qualité en ce qui concerne l'homogénéité des perforations, leur régularité dimensionnelle avec, notamment, un diamètre des perforations identiques sur les deux faces du matériau.

Toutefois, jusqu'à présent, les matériaux microperforés obtenus par ces procédés étaient toujours soit opaques, soit au mieux translucides ou rendues translucides par mouillage.

On recherche depuis maintenant longtemps des matériaux microperforés qui soient transparents et ce pour de nombreuses applications, notamment, comme membranes lorsque le matériau est un polymère flexible.

Dans le domaine de la microbiologie et en particulier des cultures cellulaires, on utilise des membranes microporeuses comme support de culture cellulaire. On recherche des membranes qui soient transparentes de manière à permettre au biologiste de contrôler la croissance des cellules par microscopie et par exemple, effectuer des colorations directes avec des colorants fluorescents, la membrane étant non fluorescente.

Les membranes microporeuses pour culture cellulaire, ainsi que pour toutes autres applications, proposées actuellement sur le marché sont toujours opaques ou au mieux translucides.

Le but de la présente invention est la réalisation d'une membrane microperforée totalement transparente à la lumière visible et aux longueurs d'ondes dans le domaine infrarouge.

Jusqu'alors, la pratique courante de l'homme du métier avait été de rechercher des membranes qui présentent un taux de surface ouverte de perforation, et par conséquent, un nombre de perforations par $cm^2$, le plus grand possible, afin d'obtenir la perméabilité la plus élevée pour un diamètre de perforation donné, le facteur limitant n'étant lié qu'à la nécessaire résistance mécanique du matériau.

En outre, dans les procédés de microperforation visés ci-dessus, on déviait le faisceau de particules accélérées de manière à balayer transversalement la bande de matériau pour régulariser la densité des perforations sur le matériau et on faisait varier l'angle d'attaque du matériau par les particules jusqu'à 45° et même jusqu'à 60° pour éviter les perforations multiples, compte tenu de l'intensité de bombardement importante.

On a tout d'abord découvert de façon fortuite en diminuant très fortement l'intensité du bombardement des particules, et partant la densité de perforation, et en diminuant dans le même temps l'angle incident d'attaque du faisceau de particules sur le matériau, que l'on maintenait une transparence totale du matériau aux rayonnements du spectre visible et infrarouge tout en conservant une perméabilité suffisante du matériau.

Les expérimentations ont, en outre, montré que pour chaque diamètre de perforation à réaliser, et pour une inclinaison moyenne de perforation donnée, il était nécessaire de ne pas dépasser une densité de perforation maximale donnée pour conserver la transparence du matériau.

Ces conditions reviennent, en fait, à ce que la distance moyenne entre deux perforations immédiatement voisines soit toujours suffisamment grande, et ce sur chacune des faces du matériau.

Les membranes jusqu'alors proposées avaient des distances minimales moyennes entre perforations de l'ordre de 1 µm et plutôt 0,5 µm.

On a découvert selon la présente invention que la distance moyenne entre deux perforations immédiatement voisines doit être d'au moins 5 µm, de préférence 7 µm, pour obtenir une transparence totale aux rayonnements du spectre visible et infrarouge proche et moyen.

La figure 1 représente une vue en coupe de deux perforations voisines. Alpha représente l'angle d'inclinaison par rapport à la perpendiculaire à la feuille du matériau, d est le diamètre des perforations, l et l' sont les distances entre les perforations respectivement sur chacune des faces du matériau. Afin de conserver la perméabilité la plus élevée possible, il est souhaitable pour un diamètre de perforation donné que la densité de perforation soit la plus élevée possible, et partant les distances entre perforations les plus petites possible.

Mais si, en outre, les distances entre perforations voisines sur chaque face, c'est-à-dire l et l', doivent être supérieures à une valeur donnée, comme on l'a vu, pour maintenir la transparence du matériau, alors la densité maximum de perforations possible est obtenue, selon l'invention, en diminuant le plus possible l'angle d'inclinaison des perforations par rapport à la perpendiculaire à la feuille du matériau.

La présente invention a donc pour objet des matériaux solides en feuille perforée transparents à un rayonnement de longueurs d'ondes dans le domaine du visible ou de l'infrarouge, dont la caractéristique essentielle est qu'ils sont constitués d'un matériau transparent lorsqu'il est à l'état non perforé et dont la distance moyenne entre deux perforations immédiatement voisines sur ses deux faces, est d'au moins 5 µm, de préférence 7 µm.

En pratique, cette distance minimale de perforations immédiatement voisines sera comprise entre 5 et 1 000 µm, le plus souvent ne dépassera pas 100 µm, voire 10 µm, étant de préférence la plus petite possible comme on l'a vu, c'est-à-dire de l'ordre de 7 µm, pour obtenir la perméabilité la plus élevée possible tout en conservant une transparence totale.

Avantageusement, selon une autre caractéristique, le matériau en feuille microperforé selon l'invention est transparent à la lumière visible et infrarouge lorsque l'inclinaison moyenne des perforations dans l'épaisseur du matériau ne dépasse pas 10°, de préférence 5°, voire le plus petit possible, par rapport à la perpendiculaire à la feuille du matériau.

Les expérimentations ont permis de montrer que la transparence totale du matériau est maintenue lorsque le diamètre des perforations (d) et la densité des perforations (n = nombre de perforations/$cm^2$) sur chacune des faces du matériau, si les perforations ont une inclinaison moyenne ne dépassant pas 5° de préférence 1°, sont telles que pour un diamètre moyen de perforation donné d exprimé en micron (µm), la densité de perforation n exprimée en nombre de perforations par $cm^2$, ne dépasse pas une valeur maximale ($n_{max}$) telle que :

$$n < \left(\frac{10^4}{d + l_m}\right)^2 = n_{max}$$

où $l_m$ est la distance moyenne entre deux perforations immédiatement voisines exprimée en µm ou de préférence :

$$n < \left(\frac{10^4}{d + 7}\right)^2 = n_{max}$$

En pratique, afin de tenir compte des limites de précisions sur la densité de perforation, la densité sera fixée à 90 % des valeurs maximales données par la formule suivante :

$$n_{max} = \left(\frac{10^4}{d + 7}\right)^2$$

Lorsque l'angle moyen d'inclinaison des perforations dépasse 5° par rapport à la perpendiculaire à la feuille, il est, bien entendu, toujours possible d'obtenir des matériaux microperforés transparents conformes à la présente invention, toutefois leur perméabilité sera comparativement plus faible ; en pratique, pour des faibles diamètres de perforation, notamment inférieurs à 5 µm, il est préférable de ne pas dépasser 10°.

Lorsque l'angle moyen d'inclinaison dépasse 5°, la densité de perforations maximale à ne pas dépasser pour un diamètre de perforation donné varie aussi en fonction de l'épaisseur de la feuille de matériau et de l'angle d'inclinaison. La relation empirique entre la densité de perforations et le diamètre des perforations peut être formulée comme suit :

$$n < \left( \frac{10^4}{d + l_m + 2e \; tg\alpha} \right)^2 = n_{max}$$

où e est l'épaisseur de la feuille du matériau exprimée en µm, et alpha est l'angle moyen d'inclinaison des perforations par rapport à la perpendiculaire à la feuille ; de préférence 1m = 7µm et n = 90 % $n_{max}$

Dans un mode de réalisation particulier, le matériau est une membrane réalisée à partir d'un film polymère flexible d'une épaisseur comprise entre 0,1 et 100 µm et plus généralement entre 10 et 50 µm, les diamètres de perforations étant compris entre 0,01 et 15 µm.

En pratique, pour des diamètres de perforations variant entre 0,01 µm et 15 µm, la densité de perforations maximale à ne pas dépasser variera respectivement entre environ $2.10^6$ et $1.10^5$ perforations/$cm^2$.

La perméabilité obtenue exprimée en pourcentage de surface ouverte de perforations sera, selon l'invention, avantageusement comprise entre 0,01 et 15 %.

L'invention est particulièrement avantageuse en ce qui concerne les petits diamètres de perforation, notamment inférieurs à 5 µm, pour lesquels les membranes proposées jusqu'alors étaient particulièrement opaques, dans ce cas il est très avantageux d'avoir recours aux petits-angles d'inclinaison de perforations, notamment inférieurs à 10°, de préférence inférieurs à 5°, pour obtenir des membranes transparentes à perméabilité suffisante. On notera, en outre, que les diamètres inférieurs à 5 µm correspondent justement aux applications où les besoins de membranes transparentes sont les plus importants.

On peut citer comme matériau utile selon l'invention le matériau synthétique, notamment, polymère néosynthétique flexible transparent choisi notamment parmi les polyesters, notamment le polyéthylène téréphtalate, les polycarbonates, notamment de bisphénol-A, les polyéthers aromatique, les polysulfones et les polyoléfines, les polyacrylates, les polyamides, les acétates et les nitrates de cellulose.

Le champ d'application des membranes microporeuses et microperforée est très vaste, mais se trouve considérablement élargi en ce qui concerne les membranes microperforées transparentes selon l'invention.

On peut citer entre autres, mais de manière non limitative, toutes les applications mettant en jeu des membranes-support pour une lecture au microscope optique ou une spectroscopie infrarouge.

En outre, les membranes transparentes obtenues selon l'invention peuvent être suffisamment perméables pour être utilisées à titre de membrane de filtration, par exemple pour certaines applications. Par ailleurs, comme on l'a vu, les matériaux selon l'invention peuvent être utiles à titre de membrane support pour culture cellulaire et sont même plus performantes pour la culture de cellules que les membranes microporeuses utilisées jusqu'à présent pour ce genre d'application.

Les membranes selon l'invention pourront recevoir un traitement de surface tel qu'un traitement chimique, par exemple d'oxydation, ou encore un traitement au polyvinylpyrrolidone pour les rendre hydrophiles, ou un traitement radiochimique du type par décharge couronne ou par plasma, ou encore un traitement photochimique, par exemple des rayonnements UV ou gamma.

Avantageusement, les membranes-support de culture cellulaire selon l'invention subissent un traitement de surface approprié favorisant l'adhésion, la croissance et la différenciation cellulaire ou un traitement contrôlant le niveau de fixation de protéines non spécifiques.

La présente invention a également pour objet un procédé dans lequel on perfore un matériau transparent à l'état non perforé, le procédé étant caractérisé en ce que la distance moyenne entre deux perforations immédiatement voisines est d'au moins 5 µm, de préférence 7 µm, sur chacune des faces du matériau.

Comme on l'a vu, dans le procédé selon l'invention, de préférence, l'inclinaison ne dépasse pas 10°, de préférence le plus petit possible, et la relation entre le diamètre des perforations et la densité des perforations est telle que caractérisée précédemment.

Les procédés d'obtention d'un matériau solide en feuille perforé décrits dans l'état de la technique, notamment, dans les documents cités ci-dessus sont applicables dans le procédé selon l'invention dans la mesure où celui-ci peut être mis en oeuvre en ayant recours à tout moyen de microperforation.

Dans un moyen de réalisation particulièrement approprié, on soumet tout d'abord le matériau à un bombardement de particules énergétiques, par exemple d'ions lourds. Chaque impact de particules sur le matériau engendre une trace d'endommagement. La densité de perforations, liée à l'intensité du bombardement, est précisemment égale à la densité d'impact. Puis, on soumet le matériau à un traitement chimique de manière à attaquer les traces d'endommagement créées par le bombardement et obtenir des perforations de diamètre désiré.

Le bombardement de particules peut être effectué, par exemple, au moyen d'un réacteur nucléaire ou de toute autre source de particules énergétiques, mais de préférence au moyen d'un accélérateur de particules.

Le cas échéant, il sera tel que l'angle moyen d'incidence du faisceau de particules sur le matériau ne dépasse pas 5° de préférence 1° par rapport à la perpendiculaire de la feuille du matériau d'une part et d'autre part que son intensité soit telle que la densité d'impact (n' exprimée en nombre d'impacts par $cm^2$) respecte les équations suivantes :

$$n' < \left(\frac{10^4}{d + lm}\right)^2 = n'_{max}$$

où d est le diamètre moyen des perforations exprimé en µm et $l_m$ est la distance moyenne entre deux perforations immédiatement voisines,
ou de préférence:

$$n' < \left(\frac{10^4}{d + 7}\right)^2 = n'_{max} \text{ et de préférence encore}$$

$$n' = 90\ n_{max}$$

Si l'angle d'incidence dépasse 5°, la relation devient:

$$n' < \left(\frac{10^4}{d + l_m + 2e\ tg\,\alpha}\right)^2 = n'_{max}$$

où d, $l_m$, e et alpha ont les significations données précédemment, avec de préférence lm = 7 et n' = 90 % $n'_{max}$

De manière à obtenir une qualité de perforation, notamment, en ce qui concerne l'homogénéité des perforations et leur régularité dimensionnelle, on utilisera de préférence un procédé de perforation tel que décrit dans WO 87/05850, avec un dispositif d'irradiation combiné à un cyclotron isochrone placé en amont en supprimant, le cas échéant, dans le dispositif les moyens d'inclinaison de l'angle d'attaque du faisceau.

Selon cette technique, la densité d'impact (ou densité de perforation) dépend, d'une part, de l'intensité du bombardement (Ampère ou nombre de particules émises par seconde) et, d'autre part, de la vitesse de défilement de la feuille de matériau devant le faisceau de particules.

En l'espèce, il convient de soumettre le matériau à l'intensité de bombardement la plus faible possible tout en maintenant la stabilité du dispositif d'irradiation et en tout état de cause très inférieure à celle utilisée précédemment. En revanche, la vitesse de défilement du matériau doit être accélérée par rapport aux procédés antérieurs.

Plus précisément, de manière à obtenir les densités de perforations mentionnées ci-dessus, on soumet le matériau à un bombardement d'ions lourds ionisés et accélérés, par exemple des ions d'argon, ayant une énergie de l'ordre de 1 à 10, plus généralement 5 à 6 Mev par nucléon, le faisceau de particules accélérées ayant une intensité comprise entre 10 et 30 nano ampères environ et ceci au moyen d'un dispositif d'irradiation combiné à cyclotron isochrone placé en amont. De plus, la feuille de matériau défile dans le dispositif d'irradiation à une vitesse comprise entre 30 et 50 m/minute environ.

Le diamètre de perforation désiré est obtenu par traitement chimique tel que décrit dans WO 87/05850. D'autres caractéristiques de ce procédé figurent dans la description de WO 87/05850 auquel il conviendra de se reporter.

D'autres caractéristiques et avantages de la présente invention apparaîtront à la lumière des exemples

qui suivent, illustrant l'invention sans toutefois en limiter la portée.

**EXEMPLE 1 : PREPARATION D'UNE MEMBRANE MICROPERFOREE TRANSPARENTE**

On a fabriqué une gamme de membranes microperforées à partir de films de polycarbonate de bisphénol A ou polyéthylène téréphtalate. Le procédé utilisé était celui décrit dans WO 87/05850 avec les adaptations quant à l'intensité de bombardement et à la vitesse de défilement du film polymère décrites ci-dessus.

L'angle d'attaque du faisceau sur le matériau était maintenu entre 0 et 5° de préférence 1°. On a obtenu une transparence totale à une lumière visible et à un rayonnement infrarouge indépendamment de l'épaisseur de la membrane avec des valeurs inférieures à celles données au tableau 1 ci-après correspondant à une distance moyenne entre perforations immédiatement voisines de l'ordre de 7μ.

Afin de tenir compte des limites de précision sur la densité de perforation, le bombardement du matériau doit être effectué de manière à obtenir une densité égale à 90 % des valeurs maximales reprises au tableau 1.

TABLEAU 1

DENSITE D'IMPACT OU DE PERFORATIONS MAXIMALE EN FONCTION DES TAILLES DES PERFORATIONS

| diamètre de perforation d (μm) | densité de perforation maximale ($cm^2$) | surface ouverte des perforations (%) |
|---|---|---|
| 0,01 à 0,1 | 2,00 $10^6$ | 0,002 à 0,02 |
| 0,2 | 2,00 $10^6$ | 0,06 |
| 0,4 | 1,80 $10^6$ | 0,2 |
| 0,6 | 1,70 $10^6$ | 0,5 |
| 0,8 | 1,60 $10^6$ | 0,8 |
| 1 | 1,60 $10^6$ | 1,2 |
| 2 | 1,20 $10^6$ | 3,9 |
| 3 | 1,00 $10^6$ | 7,1 |
| 5 | 7,00 $10^5$ | 13,6 |
| 8 | 4,50 $10^5$ | 22,3 |
| 10 | 3,50 $10^5$ | 27,2 |
| 12 | 2,80 $10^5$ | 31,3 |
| 15 | 2,50 $10^5$ | 35,0 |

Des valeurs appropriées seront par exemple celles données au tableau 2 suivant :

TABLEAU 2

MEMBRANE TRANSPARENTE DE POLYETHYLENE TEREPHTALATES OU POLYCARBONATE

(épaisseur 10 à 12 μ)

| Diamètre de perforation (μm) | Densité de perforation (nb. de perf./cm²) | Pourcentage de surface ouverte (%) |
|---|---|---|
| 0,2 | 2 $10^6$ | 0,06 |
| 0,4 | 1,6 $10^6$ | 0,2 |
| 0,6 | 1,6 $10^6$ | 0,45 |
| 0,8 | 1,6 $10^6$ | 0,8 |
| 1 | 1,6 $10^6$ | 1,26 |
| 2 | 9 105 | 2,8 |
| 3 | 4 $10^5$ | 2,83 |
| 5 | 4 $10^5$ | 7,85 |
| 8 | 1 $10^5$ | 5,03 |
| 10 | 1 $10^5$ | 7,85 |
| 12 | 1 $10^5$ | 11,31 |

Pour des angles d'attaques de 10° ou plus en moyenne, le procédé nécessite, pour obtenir la transparence totale, des densités et des surfaces ouvertes de perforations beaucoup plus faibles, par exemple, pour un matériau d'une épaisseur de 10 μ, on obtient les valeurs du tableau 3 suivant :

TABLEAU 3

| Taille de pores (µm) | DENSITE DE PERFORATION PAR $cm^2$ Angle d'inclinaison moyen des perforations | | | |
|---|---|---|---|---|
| | 10° | 20° | 30° | 45° |
| 0,01 à 0,1 | 1,0 $10^6$ | 5,0 $10^5$ | 3,0 $10^5$ | 1,5 $10^5$ |
| 0,1 | 1,0 $10^6$ | 5,0 $10^5$ | 3,0 $10^5$ | 1,5 $10^5$ |
| 0,2 | 9,0 $10^5$ | 5,0 $10^5$ | 3,0 $10^5$ | 1,5 $10^5$ |
| 0,4 | 8,5 $10^5$ | 4,5 $10^5$ | 3,0 $10^5$ | 1,3 $10^5$ |
| 0,6 | 8,0 $10^5$ | 4,5 $10^5$ | 2,5 $10^5$ | 1,3 $10^5$ |
| 0,8 | 8,0 $10^5$ | 4,5 $10^5$ | 2,5 $10^5$ | 1,3 $10^5$ |
| 1,0 | 7,5 $10^5$ | 4,5 $10^5$ | 2,5 $10^5$ | 1,3 $10^5$ |
| 2,0 | 6,5 $10^5$ | 4,0 $10^5$ | 2,5 $10^5$ | 1,2 $10^5$ |
| 3,0 | 5,5 $10^5$ | 3,5 $10^5$ | 2,0 $10^5$ | 1,0 $10^5$ |
| 5,0 | 4,0 $10^5$ | 3,0 $10^5$ | 2,0 $10^5$ | 1,0 $10^5$ |

**EXEMPLE 2 : MESURES D'ABSORBANCE DE DIFFERENTES MEMBRANES**

La figure 2 représente les courbes d'absorbance dans le domaine 300 à 800 nm du visible et proche infrarouge d'une membrane selon l'invention : "CYCLOPORE" en polyéthylène téréphtalate et des membranes translucides microperforées NUCLEPORE$^R$ en polycarbonate(PC) ou polyester (PE) ainsi qu'une membrane microperforée MILLIPORE$^R$ translucide.

Ces membranes avaient une épaisseur d'environ 10 µ et des diamètres de trous d'environ 0,4 µ. La surface ouverte des trous était de l'ordre de 13 % pour les membranes MILLIPORE$^R$ et NUCLEPORE$^R$ et 0,2 % pour la membrane CYCLOPORE.

Les distances moyennes entre perforations immédiatement voisines sont de l'ordre de 0,5 à 0,9 µm et la densité de perforation de l'ordre de $1.10^8$ $xcm^{-2}$ pour les membranes NUCLEPORE$^R$ et MILLIPORE$^R$ et de l'ordre de 7 µm et $1,6.10^6$ respectivement pour les membranes CYCLOPORE selon l'invention.

Les membranes NUCLEPORE$^R$ ET MILLIPORE$^R$ ont une absorbance proche de la limite d'absorbance du photomètre, alors que la membrane CYCLOPORE montre une absorbance quasiment nulle dans le visible et l'infrarouge (figure 2).

La figure 3 représente la courbe d'absorbance d'une membrane selon l'invention d'épaisseur 11 µm en polyéthylène téréphatalate qui montre que la membrane est parfaitement transparente aux rayonnements dans le domaine infrarouge 2 500 à 5 000 nm.

Les courbes des figures 2 et 3 montrent que les membranes selon l'invention sont transparentes aux longueurs d'ondes entre 350 et 5 000 nm.

**EXEMPLE 3 : MEMBRANE TRANSPARENTE COMME SUPPORT DE CULTURE CELLULAIRE**

Des cultures cellulaires à l'aide des membranes microperforées transparentes selon l'invention telle que décrite dans l'exemple 1 ont été effectuées. Les membranes transparentes avaient en outre, reçu un traitement chimique favorisant la culture cellulaire. Ce type de membrane a été testé quant à ses propriétés d'étalement, de croissance et de différenciation de cellules animales, spécialement des cellules polarisées telles que les cellules épithéliales et endothéliales.

Les membranes étaient présentées sous forme de disque de 25 mm de diamètre insérées dans des plaques de culture de tissus (plus spécialement des plaques de 6 puits standard) ; les membranes avaient des tailles de perforations de 0,45 µ ou 3 µ et leur épaisseur était dans les deux cas de 11 µ ou de 50 µ, les membranes étaient en polyéthylène téréphtalate parfaitement transparentes et non fluorescentes.

Malgré une perméabilité réduite avec des taux de surface ouverte de perforations de 0,2 % à 7 %, la perméabilité était suffisante et même parfaitement adaptée à une bonne diffusion des éléments nutritifs du milieu de culture et des produits bio-synthétisés et/ou transportés par les cellules lorsqu'on l'utilise comme support de culture.

Ainsi pour étudier les fonctions différenciées des cellules, en particulier pour les études sur cellules épithéliales, on a comparé les membranes selon l'invention, avec un support non poreux en polystyrène traité pour la culture cellulaire et une membrane microporeuse d'un insert de culture cellulaire Millicell-CM[R] de MILLIPORE, qui permet de séparer un puit de plaques de culture en deux compartiments et de travailler indépendamment sur les surfaces basales et apicales des cellules.

- Détail du protocole expérimental :

. culture de cellules MDCK (Madin Darby Coker Kidney cells d'origine épithéliale)
. milieu de culture : MEM supplémenté avec du sérum de veau Fetal 10 %
. revêtement des membranes :
  . membrane selon l'invention : aucun
  . support en polystyrène : aucun
  . membrane MILLIPORE pour Millicell-CM[R] : 400 µl de collagène de type 1 à 750 µg/ml dans l'éthanol 45 % (v/v) ; et l'évaporation; lavage avec PBS.

Les observations microscopiques par microscopie directe ou par inversion de phase ne sont possibles qu'avec les membranes transparentes selon l'invention.

En outre la croissance cellulaire est supérieure avec les membranes selon l'invention tel qu'il ressort du tableau 4 ci-après :

TABLEAU 4

| Temps | µg de protéines cellulaires/$cm^2$ | | |
|---|---|---|---|
| | Membranes transparentes selon l'invention | Polystyrène | Millicell-CM[R] |
| 0 h | 5 | 5 | 5 |
| 24 h | 20 | 20 | 25 |
| 48 h | 50 | 40 | 40 |
| 174 h | 125 | 45 | 60 |

## Revendications

**1)** Matériau solide en feuille microperforé transparent à un rayonnement de longueurs d'ondes dans le visible ou l'infrarouge, caractérisé en ce qu'il est constitué d'un matériau transparent à l'état non perforé et en ce que la distance moyenne entre deux perforations immédiatement voisines est d'au moins 5 µm, de préférence 7 µm, et ce sur chacune des faces du matériau.

**2)** Matériau selon la revendication 1, caractérisé en ce que l'inclinaison moyenne des perforations dans l'épaisseur du matériau ne dépasse pas 10°, de préférence 5°, par rapport à la perpendiculaire à la feuille du matériau.

**3)** Matériau selon l'une des revendications 1 ou 2, caractérisé en ce que lorsque l'inclinaison moyenne des perforations ne dépasse pas 5°, de préférence 1°, la densité de perforation est telle que :

$$n < \left( \frac{10^4}{d + l_m} \right)^2 = n_{max}$$

n = nombre de perforation par $cm^2$

d = diamètre moyen des perforations exprimé en micron

$l_m$ = est la distance moyenne entre deux perforations immédiatement voisines exprimée en micron.

**4)** Matériau selon la revendication 1 ou 2 caractérisé en ce que lorsque l'inclinaison moyenne des perforations dépasse 5°, la densité de perforation n est telle que :

$$n < \left( \frac{10^4}{d + l_m + 2e \operatorname{tg}\alpha} \right)^2 = n_{max} \text{ avec}$$

d = diamètre moyen des perforations exprimé en micron

lm = distance moyenne entre deux perforations immédiatement voisines exprimée en micron

e = épaisseur de la feuille de matériau

α = angle d'inclinaison moyen des perforations par rapport à la perpendiculaire à feuille de matériau.

**5)** Matériau selon la revendication 3 ou 4, caractérisé en ce que lm = 7 et de preference n = 90 % de $n_{max}$.

**6)** Matériau solide selon l'une des revendications précédentes caractérisé en ce que le diamètre des perforations varie entre 0,01 et 15 µm, la densité de perforations $n_{max}$ variant respectivement entre $2.10^6$ et $1. 10^5$ perforation par $cm^2$.

**7)** Matériau solide selon l'une des revendications précédentes caractérisé en ce que l'épaisseur de la feuille est comprise entre 0,1 et 100µ, de préférence entre 5 et 50 µ.

**8)** Matériau selon l'une des revendications précédentes, caractérisé en ce qu'il s'agit d'un matériau synthétique notamment polymère néosynthétique flexible choisi parmi les polyesters notamment le polyéthylène téréphtalate, les polycarbonates, notamment les polycarbonate de bisphénol-A, les polyéthers aromatiques, les polysulfones et les polyoléfines, les polyacrylates, les polyamides, les acétates et les nitrates de cellulose.

**9)** Matériau microperforé transparent selon l'une des revendications précédentes caractérisé en ce qu'il est constitué d'un polymère flexible selon l'une des revendications précédentes, de 10 à 50 µ d'épaisseur et de diamètre de perforations compris entre 0,01 et 15 µ.

**10)** Matériau selon la revendication 9 caractérisé en ce que le diamètre des perforations est inférieur à 5µ.

**11)** Procédé de préparation d'un matériau transparent selon l'une des revendications précédentes dans lequel on perfore un matériau transparent à l'état non perforé, caractérisé en ce que la distance moyenne entre deux perforations immédiatement voisines est d'au moins 5, de préférence 7 µm, et ce sur chacune des faces du matériau.

**12)** Procédé selon la revendication 11, dans lequel on soumet le matériau transparent à un bombardement

de particules énergétiques afin d'obtenir des traces d'endommagement du matériau dans toute son épaisseur et on soumet le matériau à un traitement chimique qui permet d'attaquer le matériau le long des traces d'endommagement afin d'obtenir des perforations de diamètre désiré.

**13)** Procédé selon la revendication 12, caractérisé en ce que l'angle incident moyen d'attaque des particules sur le matériau ne dépasse pas 10° de préférence 5° par rapport à la perpendiculaire de la feuille de matériau.

**14)** Procédé selon la revendication 12 ou 13, caractérisé en ce que le matériau est soumis à un bombardement d'ions lourds fortement ionisés et accélérés ayant une énergie de l'ordre de 1 à 10, plus généralement 5 à 6 Mev par Nucléon, le faisceau de particules accélérées ayant une intensité comprise entre 10 et 30 nano ampères et ceci au moyen d'un dispositif d'irradiation combiné à un cyclotron isochrone placé en amont, la feuille de matériau formant une bande défilant dans le dispositif d'irradiation avec une vitesse linéaire de défilement de ladite bande, notamment constante, de préférence comprise entre 30 et 50 m/minute environ.

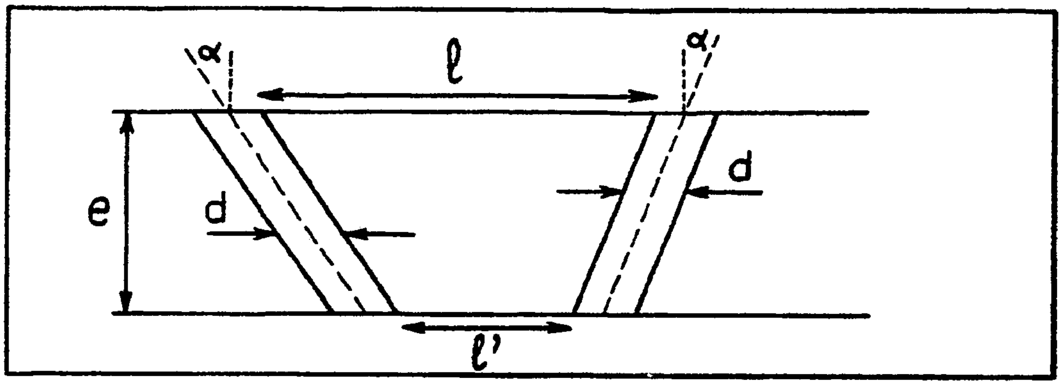


FIG_1

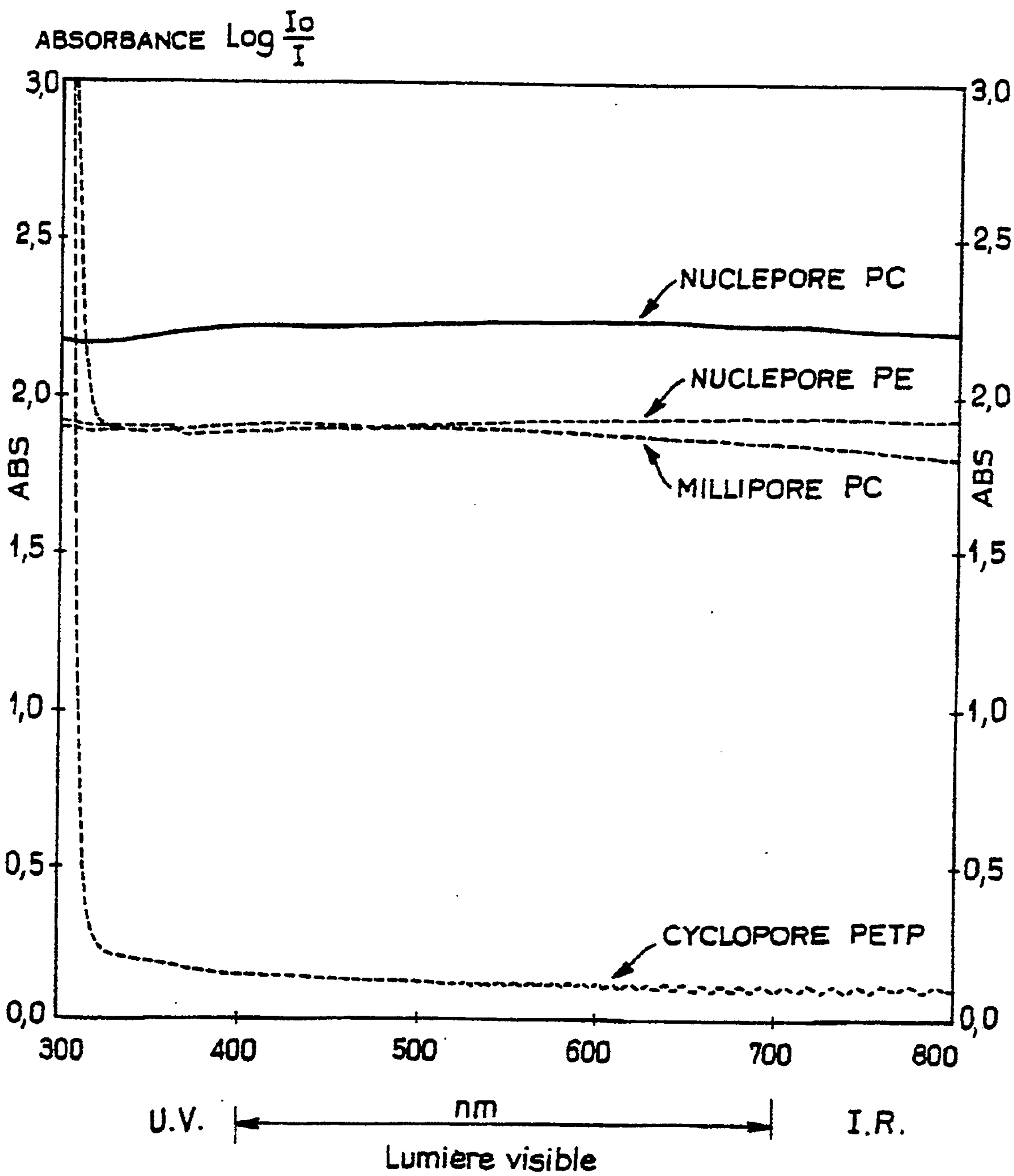
ABSORBANCE Log Io/I
3,0
2,5
2,0
1,5
1,0
0,5
0,0
ABS
NUCLEPORE PC
NUCLEPORE PE
MILLIPORE PC
CYCLOPORE PETP
300
400
500
600
700
800
U.V.
nm
Lumière visible
I.R.

FIG_2

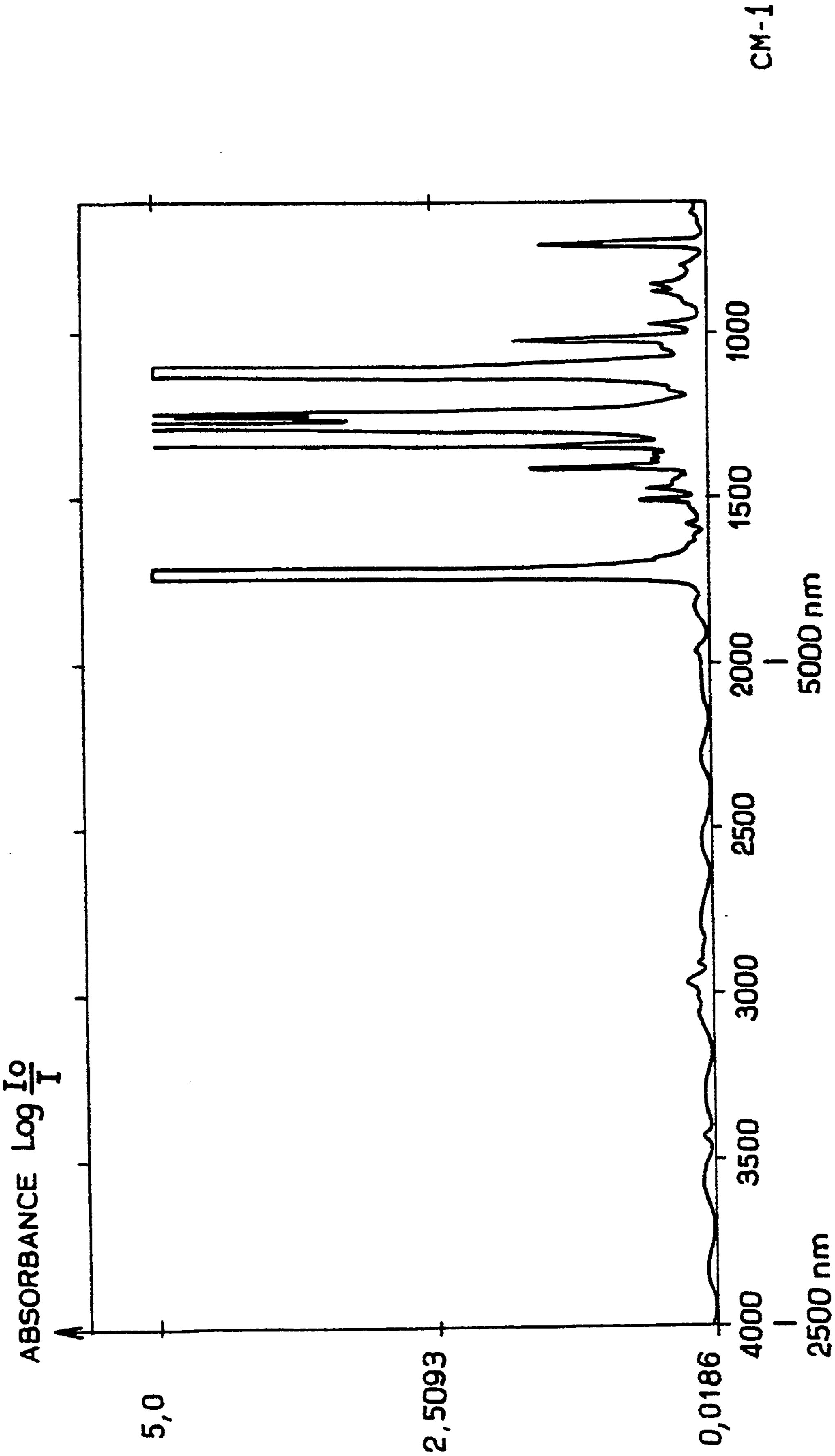
ABSORBANCE Log Io/I
5,0
2,5093
0,0186
4000
3500
3000
2500
2000
1500
1000
CM-1
2500 nm
5000 nm

FIG_3

Office européen des brevets

# RAPPORT DE RECHERCHE EUROPEENNE

Numero de la demande

EP 91 40 1379

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| Y,D | WO-A-8 705 850 (UNIVERSITE CATHOLIQUE DE LOUVAIN)<br>* Rev.; fig. * | 1-14 | B 01 D 67/00 |
| Y | CHEMICAL ENGINEERING PROGRESS, vol. 71, no. 12, décembre 1975, pages 55-61; M.C. PORTER: "Selecting the right membrane"<br>* Page 58, colonne 2; page 59, colonne 1 * | 1-14 | |
| A | EP-A-0 317 399 (COMMISSARIAT A L'ENERGIE ATOMIQUE)<br>* Page 1, ligne 48 - page 3, ligne 30 * | 1,2,6,8-14 | |
| A | EP-A-0 325 752 (AKZO N.V.)<br>* Revendications 1,8; ex.; page 3, lignes 15-25; figure 6 * | 1,2,6-9 | |
| | | | DOMAINES TECHNIQUES RECHERCHES (Int. Cl.5) |
| | | | B 01 D |

Le présent rapport a été établi pour toutes les revendications

| Lieu de la recherche | Date d'achèvement de la recherche | Examinateur |
|---|---|---|
| LA HAYE | 02-08-1991 | COUCKE A.O.M. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)